## Europäisches Patentamt

## European Patent Office

## Office européen des brevets

(11) Publication number: **0 067 038**
**B1**

# EUROPEAN PATENT SPECIFICATION

(45) Date of publication of patent specification: **28.08.85**

(51) Int. Cl.⁴: **C 07 G 11/00, C 12 P 21/00**

(21) Application number: **82302857.6**

(22) Date of filing: **03.06.82**

(54) **High molecular antitumor antibiotics AN-7D.**

(30) Priority: **10.06.81 JP 89316/81**

(43) Date of publication of application:
**15.12.82 Bulletin 82/50**

(45) Publication of the grant of the patent:
**28.08.85 Bulletin 85/35**

(84) Designated Contracting States:
**DE FR GB**

(56) References cited:

**None**

(73) Proprietor: **AJINOMOTO CO., INC.**
**5-8, Kyobashi 1-chome, Chuo-ku**
**Tokyo 104 (JP)**

(72) Inventor: **Udaka, Shigezo**
**No. 1-24-3, Uezono-cho Meito-ku**
**Nagoya-shi Aichi-ken (JP)**
Inventor: **Miyashiro, Shigeyoshi**
**No. 1-61, Mitsuke-cho Chikusaku-ku**
**Nagoya-shi Aichi-ken (JP)**
· Inventor: **Shimizu, Eikou**
**No. 1155-2, Nakamaruko Nakahara-ku**
**Kawasaki-shi Kanagawa-ken (JP)**
Inventor: **Shiio, Tsuyoshi**
**No. 1495-5, Yamazaki**
**Kamakura-shi Kanagawa-ken (JP)**

(74) Representative: **Bond, Bentley George et al**
**HASELTINE LAKE & CO. Hazlitt House 28**
**Southampton Buildings Chancery Lane**
**London, WC2A 1AT (GB)**

Courier Press, Leamington Spa, England.

**Description**

The present invention relates to newly found high molecular antitumor antibiotics AN-7D having a broad antitumor activity against cells of mouse leukemia and an extremely low toxicity.

There are known many high molecular antitumor antibiotics (which is a general term for antitumor agents), these antibiotics being high molecular peptides produced by *Actinomycetes*. Among these known antitumor agents are high molecular antitumor antibiotics having antimicrobial activities, as given in the following Table 1.

TABLE 1

Known high molecular antitumor antibiotics

| Name | Reference |
|---|---|
| Carcinocidin | Y. Harada, T. Nara and F. Okamoto: J. Antibiotics, serA 9(1), 6(1956). |
| Melanomycin | R. Sugawara, A. Mutsunae and T. Hata: J. Antibiotics, serA 10(4), 133(1956). |
| A-280 | Y. Sekizawa, S. Inouye and K. Kagino: J. Antibiotics, serA 15(6), 236(1962). |
| Iyomycin complex | S. Nomura, H. Yamamoto, A. Matsumae and T. Hata: J. Antibiotics, serA 17(3), 104(1964). |
| Plurallin | H. Ogawara, K. Maeda, K. Nitta, Y. Okami: J. Antibiotics, serA 19(1), 1(1966). |
| Actinoxanthin | Khokhlov. A. S. et. al: J. Antibiotics, 22, 541(1969). |
| Neocarzinostatin | Ishida, N., K. Mizazaki, K. Kumagai & M. Rikimaru: J. Antibiotics, serA 18(2), 68(1965). |
| Macromomycin | Chimura, H., M. Ishizuka, S. Hori, K. Kimura, J. Iwanaga, T. Takeuchi and H. Umezawa: J. Antibiotics, 21, 44(1968). |
| Sporamycin | Umeyawa, H., K. Komiyama, H. Takesima, J. Awaga & S. Omura: J. Antibiotics, 29, 1249(1976). |

Among these known antitumor antibiotics, Neocarzinostatin (hereinafter referred to as NCS) has been commercialized and now put on the market as a cancer chemotherapeutic agent. However, many of them have not yet been commercialized because of their strong toxicity and low effectiveness for human cancer.

The present invention provides high molecular antitumor antibiotics AN-7D.

High molecular antitumor antibiotics AN-7D having a broad antitumor activity against mouse leukemia and an extremely low toxicity have been found in, and obtained from, a culture broth of *Actinomycetes* belonging to the genus *Streptomyces*. The antibiotic AN-7D purified from the culture broth by a series of conventional purifying techniques which are generally employed in purification of proteins and peptides has the following chemical and physical characteristics;

(a) Molecular weight:
   12400 (estimated by Bio-Gel P-30 gel filtration method)
   12500 (by SDS-polyacrylamide gel electrophoresis method)

(b) Appearance:
   white amorphous powder;

(c) Coloration reaction:
   positive to ninhydrin and biurete reaction, negative to anthrone and Blix reaction;

(d) Solubility:
   soluble in water, insoluble in ethanol, acetone, propanol, butanol;

**0 067 038**

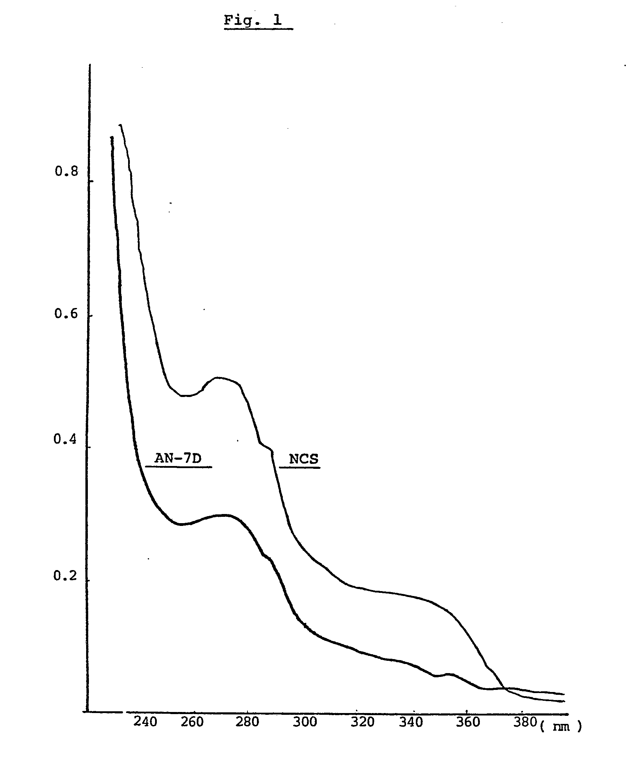

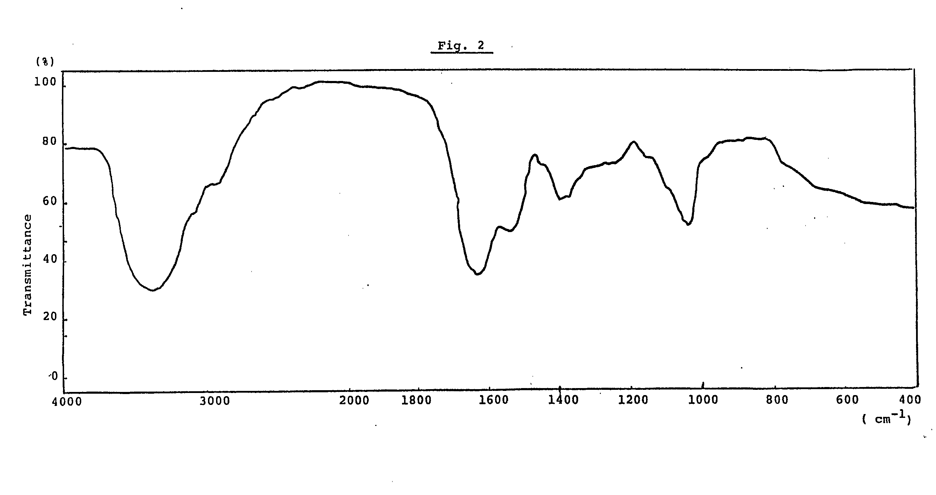

(e) Ultraviolet spectrum:
    as shown in Figure 1;
(f) Infrared spectrum:
    as shown in Figure 2.
(g) Elemental analysis:
    C: 46~47%, H: 6.0~7.0%, N: 12~13%;
(h) Composition of amino acids:
    as shown in Table 5;
(i) Isoelectric point:
    3.5 (estimated by isoelectric point electrophoresis method);
(j) Antimicrobial activity:
    Positive against *Bacillus subtilis* ATCC 6633, *Sarcina lutea* ATCC 9341 and *Streptococcus aureus* FDA 209P;
(k) Antitumor activity:
    positive against mouse leukemia L1210, P388 and P1534

AN-7D as above may be classified as a high molecular antitumor antibiotics and differs from the known antitumor antibiotics shown in Table 1 in the characteristics as shown in the following Table 2.

TABLE 2

| Antitumor antibiotics | Differences in characteristics |
| --- | --- |
| Carcinocidin | insoluble in water, chromoprotein |
| Melanomycin | insoluble in water, chromoprotein |
| A-280 | chromoprotein |
| Iyomycin complex | insoluble in water at pH 2 to 4, chromoprotein |
| Plurallin | chromoprotein, M.W. is 30,000 to 60,000 |
| Antinoxanthin | amino acids composition, methionine is not contained. |
| Neocarzinostatin | M.W. is 10,700, amino acids composition |
| Macromomycin | amino acids composition, methionin and arginine are not contained. |
| Sporamycin | amino acids composition, histidine, arginine methionine, cysteine and proline are not contained. |

Among these known agents, NCS which is reported to have a notable antitumor activity against mouse leukemia L1210 and to show very high chemotherapeutic coefficient has been commercialized as a cancer themotherapeutic agent for human cancer. Compared with NCS, AN-7D can inhibit the cell growth of mouse leukemia P1534 on which NCS is ineffective in an in vivo tumor screening system, and show higher life prolongation effect for mouse leukemia, and its maximum life prolongation effect reaches 306.4%. Therefore, AN-7D is expected to be more useful as an anticancer agent for human cancer.

AN-7D is determined by bio-assay method using *Escherichia coli* MP-2 FERM-P 5434 as described in Agric. Biol. Chem., 43 371(1979), and 1.0 unit/ml of antimicrobial activity corresponds to 10.1 µg/ml of purified AN-7D.

AN-7D is produced by culturing microorganisms belonging to the genus *Streptomyces* and capable of producing AN-7D, for example *Streptomyces griseoincarnatus* B19C (AJ 9421) (FERM BP-129) in a culture medium, and isolating and purifying the accumulated AN-7D from the culture broth.

The strain FERM BP-129 was originally deposited under the number FERM-P 6012 on the 6th June 1981 at the Fermentation Research Institute, Agency of Industrial Science and Technology, Ministry of International Trade and Industry (FRI), 1—3, Higashi 1-chome, Yatabe-machi, Tsukuba-gun, Ibaraki-ken 305, Japan. This deposit was converted to a deposit under the Budapest Treaty on the 14th May 1982 when the FRI acquired the status of an International Depository Authority as from the 2nd May 1981.

The strain B19C has the same general characteristics as the known strain ATCC 23623, which

3

characteristics are described in Int. J. Syst. Bact. 18:20 and 328(1968), and in addition the ability to produce AN-7D. The strain B19C was isolated from soil obtained from Mount Hakone, near Mount Fuji, Kanagawa-ken, Japan.

The microorganisms as above can be cultured aerobically in a conventional culture medium containing carbon sources, nitrogen sources, inorganic ions, and when required, minor nutrient elements, preferably with adjusting pH 4 to 9 and 25 to 38°C for 1 to 5 days.

Suitable carbon sources include starch, saccharides such as glucose, fructose, maltose, rhamnose, xylose, lactose, sucrose and molasses and hydrolyzed starch containing these saccharides; organic acids such as acetic acid; and alcohols such as ethanol, and glycerol, which are preferably used. Suitable nitrogen sources include, for example ammonium sulfate, gaseous ammonia, ammonia water, amino acids, hydrolyzed casein and yeast extract containing amino acids. Suitable inorganic ions include, for example, $K^+$, $PO_4^{---}$, $Ca^{++}$, $Mg^{+++}$, $Cu^{++}$, $Zn^{++}$, $Mn^{++}$ and $Fe^{++}$. AN-7D produced in the culture broth can be purified and isolated from the culture broth by a series of entirely conventional purifying techniques such as salting out, dialysis, gel filtration, ion-exchanging chromatography which have been employed in purification of proteins and peptides.

The above disclosure generally describes the present invention. A more complete understanding of the invention can be obtained by reference to the following examples, which are provided herein for purposes of illustration only and are not intended to be limiting unless otherwise specified.

Example 1

Twenty ml portions of the Culture Medium A having the composition shown in Table 3 were placed into 500 ml-flasks, which were then heated at 110°C for 10 minutes for sterilization.

TABLE 3
Composition of culture medium

| Component | Conc. | Medium | |
| --- | --- | --- | --- |
| | | A | B |
| Glucose | (g/dl) | 1.0 | 3.0 |
| Soluble starch | (g/dl) | 1.0 | — |
| Peptone | (g/dl) | 0.5 | 0.5 |
| Dried yeast | (g/dl) | 0.5 | 0.5 |
| Bouillon | (g/dl) | — | 0.5 |
| NaCl | (g/dl) | 0.5 | 0.5 |
| $CaCl_2 \cdot 2H_2O$ | (g/dl) | 0.1 | 0.2 |
| $MnCl_2 \cdot 4H_2O$ | (mg/dl) | 0.1 | 2.0 |
| $FeCl_2 \cdot 6H_2O$ | (mg/dl) | 0.1 | — |
| pH | | 7.0 | 7.0 |

*Streptomyces griseoincarnatus* B19C FERM BP-129 grown on bouillon agar slant was inoculated into each batch of culture medium, and cultured with shaking at 30°C for 24 hours to prepare seed culture broth. On the other hand, three hundred ml portions of Culture Medium B in Table 3 were placed into 1.0 liter-fermentation vessels and heated for sterilization. Each medium was inoculated with 20 ml of the seed culture broth as above and cultivation was conducted at 30°C with agitation and aeration. After 50 hours of cultivation, antibacterial activity of the resultant culture broth against *Escherichia coli* MP-2 was 16 Units/ml, which corresponds with 162 µg/ml of purified AN-7D. 1.0 liter culture broth thus obtained was centrifuged at a low temperature (0—5°C) at 8,000 rpm for 20 minutes to remove microbial cells, and the supernatant was then saturated to 80% with ammonium sulfate.

After adjusting the pH to 3.2 with 6N-acetic acid solution, the solution was allowed to stand at 5°C for 30 minutes, thereby AN-7D was precipitated together with other high molecular impurities from the ammonium sulfate solution. Then the precipitate was collected by centrifugation (10,000 rpm for 20 minutes) and dissolved into 100 ml deionized water. The resulting solution was placed into Cellophane bags and dialyzed against 0.02 M phosphate buffer (pH 7.0) at 5°C for 24 hours. Then the dialyzate was

4

centrifuged (8,000 rpm) at 5°C for 10 minutes to remove impurities precipitated during the dialysis, and 40 ml of DEAE-cellulose DE-52 was added to the supernatant solution, which was stirred in an ice bath for 30 minutes. The mixture was filtered with a filter paper and the filtrate of which pH was adjusted to 5.0 with 2N-acetic acid was dialyzed at 5°C against 0.002 M phosphate buffer (pH 7.0) for 24 hours and applied to a column of DEAE-cellulose DE-52 (1.6×40 cm) equilibrated with 0.002 M phosphate buffer (pH 7.2). The column was washed with a sufficient volume of the equilibration buffer, and then eluted with the same buffer containing 0.2 M sodium chloride.

Fractions with the antibacterial activity were collected and lyophilized to obtain 40.5 mg white powder. The active fractions after DEAE-cellulose chromatography were submitted to gel filtration on a column of Bio-Gel P-30 (1.6×40 cm) using 0.002 M phosphate buffer containing 0.1 M sodium chloride as a solvent at a flow rate of 1.0 ml/5 min.

The effluents with the antibacterial activity were collected, dialyzed against deionized water at 5°C for 48 hours and lyohilized to obtain 20 mg AN-7D in the form of white powder.

AN-7D thus purified has 6225 units/mg of the antibacterial activity to *E. coli* MP-2 and was identified to be homogeneous both on Bio-Gel P-30 gel filtration and polyacrylamide gel electrophoresis.

Chemical and physical characteristics of AN-7D thus purified were determined and the results are as follows:

(a) Molecular weight:
    12400 (estimated by gel filtration)
    12500 (by SDS-polyacrylamide gel electrophoresis)

In the estimation of Molecular weight by gel filtration method, the sample was submitted to gel filtration on a column of Bio-Gel P-30 (1.6×20 cm) using 0.02 M phosphate buffer (pH 7.2) containing 0.1 M sodium chloride as a solvent with flow rate of 3.0 ml/5 min. and the molecular weight was estimated from the reference curve of the standard molecular markers. Another estimation of the molecular weight was performed by SDS-polyacrylamide gel electrophoresis using 30% polyacrylamide gel according to the method of Weber et al as described in J. Biol. Chem., vol. 1, 244, 16, 4406(1969).

(b) Coloration reaction:
    as shown in the following Table 4.

TABLE 4
Coloration reaction

| Reaction | Result |
|---|---|
| ninhydrin reaction | + |
| biuret reaction | + |
| anthrone reaction | − |
| Blix reaction* | − |

\* As the pretreatment, sample was hydrolyzed with 4N-HCl at 100°C for 4 hours to liberate hexosamine.

(c) Ultraviolet spectrum:
    as shown in Figure 1;
(d) Amino acids composition:
    as shown in Table 5;

**0 067 038**

TABLE 5
Composition of amino acid

| Amino acid | % | Amino acid | % |
|---|---|---|---|
| Asp | 12.0 | Met | 0.2 |
| Thr | 10.1 | Ile | 2.2 |
| Ser | 7.2 | Leu | 6.6 |
| Glu | 7.1 | Tyr | 1.9 |
| Pro | 4.6 | Phe | 5.4 |
| Gly | 8.1 | Lys | 2.0 |
| Ala | 10.3 | His | 0.9 |
| Cys | —* | Trp | —* |
| Val | 8.1 | Arg | 4.0 |
| NH₃ | 7.8 | | |

\* The content was not determined.

The result given in Table 5 was determined by amino acid autoanalyzer (Model KLA 5-B, Hitachi Seisakusho, Tokyo)

(e) Solubility:

AN-7D is soluble in water but insoluble in ethanol, acetone, butanol and propanol.

(f) Infrared spectrum:

as shown in Table 2.

(g) Elemental analysis:

C: 46~47%, H: 6.0~7.0%, N: 12~13%;

(h) Isoelectric point:

3.5 (determined by electrofocusing Apparatus of model SJ-1071, Atto Co., Inc., equipped with Servalyt Precotes)

In this experiment, isoelectric points of ferritin, bovine albumin, and β-lactoglobulin were also determined as the control, which were found to be 4.4, 4.7, and 5.3 respectively.

(i) Antibacterial activity:

as shown in Table 6;

6

TABLE 6
Minimum inhibition concentration
(MIC) of AN-7D

| Test strain | MIC (µg/ml) | |
|---|---|---|
| | AN-7D | Neocarziostatin |
| *Escherichia coli*<br>K-12 ATCC 10798 | >100 | >100 |
| *Escherichia coli*<br>MP-2 | 4 | 2 |
| *Bacillus subtilis*<br>ATCC 6633 | 3 | 15 |
| *Sarcina lutia*<br>ATCC 9341 | 1 | 2 |
| *Pseudomonas aeruginosa*<br>ATCC 10145 | >100 | >100 |
| *Staphylococcus aureus*<br>FDA 209P | 20 | 10 |

MIC in Table 6 was determined by the following procedure;

Two ml portions of M$_3$-Medium (Bactoantibiotic Medium-3, Difco, 1.75%, pH 7.2) were placed into test tubes and heated 120°C for 10 minutes. Then each test strain in Table 6 was inoculated into each batch of culture medium, and cultured with shaking at 37°C for 20 hours to obtain seed culture broths. Then each 0.05 ml of the culture broth was inoculated into 20 ml of M$_3$-Medium containing AN-7D or NCS and cultured at 37°C for 20 hours with shaking. Thereafter, optical density of the resultant culture broth at 660 nm was determined, and MIC was calculated.

(j) Antitumor activity:
as shown in Table 7

TABLE 7
Degree of multiplication of L1210 cells (%)

| Concentration<br>(µg/ml) | AN-7D | NCS |
|---|---|---|
| 0 | 100 | 100 |
| 0.05 | 100 | 100 |
| 0.10 | 100 | 100 |
| 0.20 | 100 | 100 |
| 0.30 | 0 | 85 |
| 0.50 | 0 | 20 |
| 1.00 | 0 | 0 |

The results given in Table 7 were obtained by the following procedure; 10.38 g of RPMI 1640 medium (Moore, G. E., et al., J. Nat. Cancer Inst., 36, 405(1966), Flow Laboratories Inc., U.S.A.) was dissolved into 1.0 liter distilled water and 1.0 g NaHCO$_3$ was added to it. Then the solution was filtrated with Millipore filter (pore size: 0.22 µ) and supplemented with 100 ml of germ-free blood serum. 1.0 ml portions of the culture medium thus prepared were placed into Falcon 3047 24-well Multi Well (Becton Dickinson & Co.) aseptically. Then 0.05 ml culture of mouse leukemia L1210 cells previously cultured was inoculated into each batch of the culture medium, and the definite amount of purified AN-7D or NCS dissolved in the same

7

medium was added simultaneously. Thereafter, they were incubated in $CO_2$ gas incubator (concentration of $CO_2$ gas: 7.0%) at 30°C. After 5 days incubation, the numbers of multiplicated cells were counted under handstanding microscope and the relative multiplication number is given in Table 7.

Example 2

$1 \times 10^5$ cells of mouse leukemia L1210 were transplanted intraperitoneally into $BDF_1$ female mice bred for 5 weeks.

From the next day after transplantation of tumor cells, 0.1~39 mg/kg/day of purified AN-7D were administrated intraperitoneally for 5 days, and average survival days were determined, from which life prolongation effects were calculated.

In the similar manner, life prolongation effect of commercial NCS were determined, and the results are also shown in Table 8.

TABLE 8
Antitumor activity on mouse leukemia

| Amount of administration (mg/kg) | AN-7D | | NCS | |
|---|---|---|---|---|
| | A.S.D.* | L.P.E.** | A.S.D. | L.P.E. |
| 0 (control | 9.4±0.5 | 100 | 9.4±0.5 | 100 |
| 0.1 | | | 13.4±1.8 | 142.6 |
| 0.25 | | | 14.2±2.9 | 151.1 |
| 0.63 | | | 9.2±0.4 | 97.9 |
| 1.0 | 10.6±1.1 | 112.8 | | |
| 1.56 | | | 7.2±0.4 | 76.6 |
| 2.5 | 14.0±4.1 | 148.9 | | |
| 6.3 | 15.0±2.2 | 159.6 | | |
| 15.6 | 16.8±5.9 | 178.7 | | |
| 39 | 28.8±15.3 | 306.4 | | |

\*: Average survival days
\*\*: Life prolongation effect

Example 3

$1 \times 10^6$ cells of mouse leukemia P388 were transplanted intraperitoneally into $BDF_1$ female mice bred for 9 weeks. Then 10 and 20 mg/kg/day of purified AN-7D were administrated for 5 days intraperitoneally.

In a similar manner as above, $1 \times 10^5$ cells of mouse leukemia P1534 were transplanted into $BDF_1$ mice.

From the next day after transplantation of tumor cells, 10 and 25 mg/kg/day of the purified AN-7D were administrated to the mice for 5 days intraperitoneally. Then life prolongation effect was calculated from the average survival days of test groups and the results obtained are shown in Table 9 and Table 10.

8

# 0 067 038

### TABLE 9
### Life prolongation effect on mouse
### leukemia P388

| Amount of administration (mg/kg) | Average survival days | Life prolongation effect (%) |
|---|---|---|
| none | 10.4±2.1 | 100 |
| 10 | 15.6±3.0 | 150 |
| 25 | 14.0±0 | 134 |

### TABLE 10
### Life prolongation effect on mice
### leukemia P1534

| Amount of administration | Average survival days | Life prolongation effect (%) |
|---|---|---|
| none | 11.2±2.6 | 100 |
| 25 | 18.4±5.7 | 164.3* |

* Significantly different to control (P<0.05)

As shown in Table 10, administration of AN-7D can prolong the lives of the P1534 bearing mice significantly. In a similar manner, 0.25 mg/kg/day of NCS was administrated, but the life prolongation effect obtained is as low as 75%.

**Claims**

1. A high molecular antitumor antibiotic AN-7D having the following characteristics:
   (a) Molecular weight:
   12400—12500;
   (by gel-filtration and SDS-polyacrylamide gel electrophoresis methods)
   (b) Appearance:
   white amorphous powder;
   (c) Coloration reaction:
   positive to ninhydrin and biuret reaction, negative to anthrone and Blix reaction;
   (d) Solubility:
   soluble in water, insoluble in ethanol, acetone, propanol and butanol;
   (e) Ultraviolet spectrum:
   as shown in Figure 1;
   (f) Infrared spectrum:
   as shown in Figure 2;
   (g) Elemental analysis:
   C: 46~47%, H; 6.0~7.0%, N: 12~13%;
   (h) Composition of amino acids:
   Asp (12.0%), Thr (10.1%), Ser (7.2%), Glu (7.1%), Pro (4.6%), Gly (8.1%), Ala (10.3%), Val (8.1%), $NH_3$ (7.8%), Met (0.2%), Ile (2.2%), Leu (6.6%), Tyr (1.9%), Phe (5.4%), Lys (2.0%), His (0.9%), Arg (4.0%),
   (i) Isoelectric point:
   3.5 (by isoelectric point electrophoresis)
   (j) Antimicrobial activity:
   positive against *Bacillus subtilis* ATCC 6633, *Sarcina lutea* ATCC 9341 and *Streptococcus aureus* FDA 209P;
   (k) Antitumor activity:
   positive against mouse leukemia L1210, P388 and P1534.
2. A high molecular antitumor antibiotic AN-7D, obtained by culturing *Streptomyces griseoincarnatus* FERM BP-129.
3. *Streptomyces griseoincarnatus* FERM BP-129 in the form of a pure culture.

9

**0 067 038**

**Patentansprüche**

1. Hochmolekulares Antitumor-Antibiotikum AN-7D, das folgende Eigenschaften hat:
(a) Molekulargewicht:
12400 bis 12500;
(mittels der Methode der Gefiltration und der SDS-Polyacrylamidgel-Elektrophorese)
(b) Aussehen:
weißes amorphes Pulver;
(c) Farbreaktion:
positive Ninhydrin- und Biuret- Reaktion, negative Anthron- und Blix-Reaktion;
(d) Löslichkeit:
löslich in Wasser, unlöslich in Ethanol, Aceton, Propanol und Butanol;
(e) Ultraviolettspektrum:
wie in Fig. 1 gezeigt;
(f) Infrarotspektrum:
wie in Fig. 2 gezeigt;
(g) Elementaranalyse:
C: 46 bis 47%, H: 6,0 bis 7,0%, N: 12 bis 13%;
(h) Zusammensetzung aus Aminosäuren:
Asp (12,0%), Thr (10,1%), Ser (7,2 %), Glu (7,1 %), Pro (4,6 %), Gly (8,1 %), Ala (10,3 %), Val (8,1 %), NH$_3$ (7,8 %), Met (0,2 %), Ile (2,2 %), Leu (6,6 %), Tyr (1,9 %), Phe (5,4 %), Lys (2,0 %), His (0,9 %), Arg (4,0 %),
(i) Isoelektrischer Punkt:
3,5 (mittels elektrophoretischer Bestimmung des isoelektrischen Punkts);
(j) Antimikrobielle Aktivität:
positiv gegenüber Bacillus subtilis ATCC 6633, Sarcina lutea ATCC 9341 und Streptococcus aureus FDA 209P;
(k) Antitumoraktivität:
positiv gegenüber Leukämie L1210, P388 und P1534 der Maus.
2. Hochmolekulares Antitumor-Antibiotikum AN-7D, erhalten durch Züchten von Streptomyces griseoincarnatus FERM BP-129.
3. Streptomyces griseoincarnatus FERM BP-129 in Form einer Reinkultur.

**Revendications**

1. Un antibiotique AN-7D antitumoral de haut poids moléculaire ayant les caractéristiques suivantes:
(a) poids moléculaire:
12400—12500;
(selon les méthodes de filtration sur gel et d'électrophorèse sur gel de SDS-polyacrylamide);
(b) aspect:
poudre amorphe blanche;
(c) réactions colorées:
réactions à la ninhydrine et réaction du biuret positives, réaction à l'anthrone et réaction de Blix négatives;
(d) solubilité:
soluble dans l'eau, insoluble dans l'éthanol, l'acétone, le propanol et le butanol;
(e) spectre ultraviolet:
comme illustré par la figure 1;
(f) spectre infrarouge:
comme illustré par la figure 2;
(g) analyse élémentaire:
C=46~47%, H=6,0~7,0%, N=12~13%;
(h) composition des aminoacides:
Asp (12,0%), Thr (10,1%), Ser (7,2%), Glu (7,1%), Pro (4,6%), Gly (8,1%), Ala (10,3%), Val (8,1%), NH$_3$ (7,8%), Met (0,2%), Ile (2,2%), Leu (6,6%), Tyr (1,9%), Phe (5,4%), Lys (2,0%), His (0,9%), Arg (4,0%)
(i) point isoélectrique:
3,5 (par électrophorèse au point isoélectrique);
(j) activité antimicrobienne:
positive contre *Bacillus subtilis* ATCC 6633, *Sarcina lutea* ATCC 9341 et *Streptococcus aureus* FDA 209P;
(k) activité antitumorale:
positive contre les leucémies L1210, P388 et P1534 de la souris
2. Antibiotique AN-7D antitumoral de haut poids moléculaire obtenu par culture de *Streptomyces griseoincarnatus* FERM BP-129.
3. *Streptomyces griseoincarnatus* FERM BP-129 sous forme d'une culture pure.

10

Fig. 1

Fig. 2

(%)

Transmittance

0 067 038